# EUROPEAN PATENT SPECIFICATION

(11) **EP 1 128 863 B1**
(45) Date of publication and mention of the grant of the patent: **19.04.2006**
(21) Application number: 00958943.3
(22) Date of filing: 21.09.2000
(51) Int. Cl.: A61M 16/00, A61M 16/12

(54) **Improving oxygenation in subjects suffering impaired oxygenation**
Verbesserung der Sauerstoffversorgung in Lebewesen mit gestörter Sauerstoffversorgung
Amelioration de l'oxygenation chez sujets souffrant d'oxygenation déficiente

(30) Priority: 22.09.1999 US 155526 P
(43) Date of publication of application: 05.09.2001
(73) Proprietor: INSTRUMENTARIUM CORPORATION, 00510 Helsinki (FI)
(72) Inventor: HEINONEN, Erkki, FIN-00750 Helsinki (FI); MERILÄINEN, Pekka, FIN-00660 Espoo (FI); NYMAN, Görel, S-74190 Knivsta (SE); HÖGMAN, Marieann, S-74793 Alunda (SE)
(74) Representative: Gillard, Richard Edward
(86) International application number: PCT/IB2000/001343
(87) International publication number: WO 2001/021239

(56) References cited:
- EP-A- 0 834 332
- EP-A- 0 872 254
- EP-A- 0 878 208
- EP-A- 0 879 612
- WO-A-98/44976
- DE-C- 4 325 319
- US-A- 5 558 083
- US-A- 5 918 596

## Description

### BACKGROUND OF THE INVENTION

Human and animal body metabolism uses oxygen and produces carbon dioxide. The required oxygen is received from the atmospheric air during respiration, in the course of which waste carbon dioxide is released. The gas exchange between the body and the environment takes place in the lung alveoli, where pulmonary blood capillaries are separated from the gas space in the lung in communication with the atmospheric air by only a thin membrane permeable for gases. The pulmonary blood flow passing through the alveoli equilibrates in gas partial pressure with the alveolar gas, resulting in blood oxygen uptake and carbon dioxide release. During each breath the alveolar blood gas concentration is changed as a result of the oxygen supplement and carbon dioxide removal. The blood transports the oxygen from the lungs to the sites of consumption and waste carbon dioxide from the sites of metabolism back to the lungs.

Blood flow rates through the lungs and perfusion pressure are regulated by the smooth muscle tension of the pulmonary capillaries. This regulation is mediated by endothelium derived nitric oxide. Insufficient local NO production increases smooth muscle tone. This results in pulmonary vasoconstriction and impaired blood flow or, altematively, elevated pulmonary artery pressure. Pulmonary hypertension is present in various circumstances, such as pneumonia, traumatic injury, aspiration or inhalation injury, fat embolism in the lung, acidosis, inflammation of the lung, adult respiratory distress syndrome, acute pulmonary edema, acute mountain sickness, post cardiac surgery, acute pulmonary hypertension, persistent pulmonary hypertension of the newborn, prenatal aspiration syndrome, hyaline membrane disease, acute pulmonary embolism, heparin-protamine reactions, sepsis, or hypoxia (including that which may occur during one-lung anesthesia), as well as those cases of chronic pulmonary vasoconstriction which have a reversible component, such may result from chronic pulmonary hypertension, bronchopulmonary dysplasia, chronic pulmonary embolism, idiopathic or primary pulmonary hypertension, or chronic hypoxia due to chronic obstructive lung disease.

U.S. Patent 5,485,827 discloses a method using inhaled nitric oxide (NO) useful for preventing or reversing acute pulmonary vasoconstriction, such as that arising from the foregoing injuries. A method for using NO gas also to achieve bronchodilatation and thereby improve the distribution of other agents administered by inhalation is also disclosed.

U.S. Patent 5558083 discloses the use of nitric oxide for newborns having persistent pulmonary hypertension in order to increase the oxygen saturation in those infants.

A special advantage of inhaled NO as a pulmonary vasodilator is its selectivity. NO is rapidly bound with blood hemoglobin, thus the free NO needed for mediating the vasodilatation is available selectively for the smooth muscles of the pulmonary capillaries only, and even more specifically, for the pulmonary capillaries adjacent ventilated alveoli. The pulmonary blood for alveoli which are not ventilated form a pulmonary shunt flow, since the non-ventilated alveoli are rapidly equilibrated with the pulmonary artery blood gases and no further gas exchange will take place. The pulmonary blood flow not participating in the gas exchange is thus called shunt flow. One reason for using inhaled NO therapy is to reduce the alveolar-arterial oxygen partial pressure difference for better oxygenation. The mechanism for this is reduction of the shunt. Administration of NO to ventilated alveoli dilates the pulmonary capillaries carrying blood for gas exchange. Capillaries in communication with the non-ventilated alveoli are constricted due to the low NO concentration. This results in blood perfusion redistribution towards the ventilated lung areas. When the portion of the pulmonary perfusion participating in the blood flow increases, the arterial oxygen partial pressure will increase, improving oxygenation.

Despite this well known mechanism, the published research results of inhaled NO for improving oxygenation have been limited. Examples of studies of oxygenation improvements are e.g. Gerlach et al.: "*Long-term inhalation with evaluated low doses of nitric oxide for selective improvement of oxygenation in patients with adult respiratory distress syndrome*", Intensive Care Med (1993) 19:443-449; Gerlach et al.: "*Time-course and dose-response of nitric oxide inhalation for systemic oxygenation and pulmonary hypertension in patients with adult respiratory distress syndrome*", Euro J. of Clinical Investigation (1993) 23: 449-502:, Benzing et al.: "*Hypoxic pulmonary vasoconstriction in non-ventilated lung areas contributes to differences in hemodynamic and gas exchange responses to inhalation of nitric oxide*", Anesthesiology (1997) 86: 1254-61. In all these, and other, published studies, NO has been administered to patients having a diagnosis of lung disease.

The NO delivery rate for improving oxygenation has both minimum and maximum limits making the oxygenation improvement clinically challenging. The loss of the oxygenation effect with increased doses is most likely traced back to the smooth muscle sensitivity. With increasing delivery, more NO diffuses to non- or poorly ventilated alveoli causing dilatation. This impairs the improvement in oxygenation seen prior to increasing the dose, as discussed by Gerlach in "*Time-course*...." The balance between improved and impaired gas exchange depends on lung status and is, therefore, individual for each patient. When the ventilation or lung performance is changing, most likely this balance is also affected.

Pulmonary shunt variation is very commonly present in healthy and sick lungs in various daily life and treatment conditions. Atelectasis, areas of the lung not participating in the gas exchange due to collapse of the alveoli, prevent normal oxygen delivery, and increases the pulmonary shunt. It has been pointed out that atelectasis is present during almost every anaesthesia (A. Strandberg et al: "*Atelectasis during anaesthesia and in the postoperative period*", Acta Anaesthesiol. Scand. (Feb. 1986) 30:2, 154-8); L. Tokic et al: "*Lung collapse and gas exchange during general anesthesia: effects of spontaneous breathing, muscle paralysis, and positive end expiratory pressure*", Anesthesiology (Feb. 1997) 66:2, 157-67). In normal healthy subjects this atelectasis is not very significant due to the oxygenation reserve.

The severity of atelectasis will increase along with decrement of the oxygenation reserve. During artificial ventilation in anaesthesia and intensive care it is possible to increase the inhaled oxygen fraction and thereby increase the oxygenation reserve. In extensive collapse of lung, aeration with even 100% oxygen in the inhaled gases may not be sufficient An example where the oxygenation reserve is endangered is horses experiencing anaesthesia in the unnatural supine position. The lungs, anatomically suited for the standing position, will be compressed by the body mass in the supine position. The lung volume can be reduced by as much as 50% and cause a pulmonary perfusion shunt of 20-50%. NO delivered to the inspired gas can redistribute the blood flow to ventilated areas and improve oxygenation.

Similar problems, which may in the worst case be chronic in nature, are encountered by humans having morbid obesity, i.e. twice the normal body weight, or 50 kg over the normal, or a body mass index over 40. In the supine position the lung functional residual capacity, FRC, is markedly reduced by the tissue mass restricting the lung volume. This may lead to impaired oxygenation and pulmonary shunt without any diagnosis of lung disease especially when sleeping when the lungs are squeezed by the body mass. Even worse, the diaphragm of obese people tends to assume a position which can be described as elevated when a person is standing, leading to a decrease in lung volume and increase in shunt. This may cause oxygenation problems even in normal daily life. The problem also occurs in anaesthesia or intensive care, and extends also to postoperative care where the restoration of normal pulmonary functions may take 4-5 hours (Brodsky: "*Morbid obesity*", Current Anaesthesia and Critical Care (1998) 9:249-254).

The compression of the lungs by the mass exerted on it may cause alveolar collapse during the time in which expiration occurs. The collapsed alveoli will open during the course of an inspiration when the lung opening pressure increases. The lung opening pressure required to open the lung increases towards the lowermost lung regions where the presence of the compressing mass of an obese person also increases, and more lung volume will be recruited along the progress of the inspiration. In spontaneous breathing this opening pressure is an underpressure in pleural cavity generated by breathing muscles of which the diaphragm is the most important. In artificial ventilation the opening pressure is overpressure in the lung gas space generated by the ventilator. At the beginning of expiration the lung opening pressure is relieved and the emptying of the lungs starts. The lung regions opened last during inspiration will close first at the beginning of expiration. This lung collapse will continue upwards from the lowermost lung region during expiration.

Due to the high diffusion capacity of alveolar NO into blood and the sensitivity of the capillary smooth muscle tone to the vasodilatory effect of NO, the NO has a rapid effect on the smooth muscle. Even the short period at the end of inspiration when the last alveoli will be opened before recollapse or bronchial reclosure at the beginning of expiration may be enough to dilate the capillaries. In the collapsed alveoli, the perfusion so enhanced does not participate in the gas exchange. The capillaries around the alveoli remaining open throughout the expiration will also dilate due to the inhaled NO. If the increased oxygenation in the latter is enough to overcome the ineffective dilatation around the collapsed alveoli a positive net oxygenation improvement will be obtained.

### SUMMARY OF THE INVENTION

The current invention concerns the improvement of oxygenation in subjects having essentially healthy lungs, as evidenced by the absence of a diagnosis of lung disease or injury, but having reduced alveolar gas exchange area. This reduction may be caused by such acute circumstances as an unnatural body position, or may be, for example, chromic as caused by obesity.

According to the invention, there is provided use of NO gas for the preparation of a breathable gas for improving oxygenation and reducing shunt perfusion by providing NO gas into ventilated alveoli of the lungs in a subject having no diagnosis of lung injury but suffering impaired oxygenation, the lungs of the subject having a ventilated portion of alveoli that remain open during inspiration and expiration and a further alveolar portion comprising one in which the alveoli collapse during expiration as a result of compression by tissue mass, wherein the step of providing NO gas to the lungs of the subject is carried out in such a manner that a sufficient amount of NO is delivered to the ventilated alveolar portion to cause capillary vasodilatation while providing an amount of NO to the further alveolar portion which is small compared to that delivered to said ventilated alveolar portion. NO provided to alveoli collapsing during expiration is small compared to those remaining open, thereby to provide net reduction in the shunt and thus an oxygenation improvement This result may be gained either by precise control of the inspired NO concentration or by pulsed administranon of the NO.

The inhalation NO delivery to the collapsing alveoli has to be small enough not to exert vasodilatation, whereas the delivery to the alveoli remaining open throughout inspiration and expiration has to be sufficient to create the dilatation. For an administration of NO taking place at constant inspired concentration, precise control of the delivery rate is required to limit the amount of NO delivered to collapsing lung areas yet to provide enough NO for the alveoli remaining open so as to get the net effect in the form of pulmonary shunt reduction and oxygenation improvement.

Alternatively, the pulse NO administration can be timed to occur in the first e.g. 30-70% of inspiration. Such administration avoids delivery into the last opening alveoli and thus dilatation of the capillaries associated with those alveoli. With pulsed administration, control of the NO delivery rate is less critical.

Various other features, objects, and advantages of the invention will be made apparent from the following detailed description and the drawings.

### BRIEF DESCRIPTION OF THE DRAWING

Fig. 1 is a schematic diagram of apparatus suitable for carrying out the method of the present invention;
Fig. 2 is a graph relating lung volume to intrapleural pressure;
Fig. 3 is a simplified showing of the information contained in the graph of Fig. 2;
Fig. 4 and is a schematic diagram of alternative apparatus for carrying out the method of the present invention.

### DETAILED DESCRIPTION OF THE PREFERRED EMBODIMENT

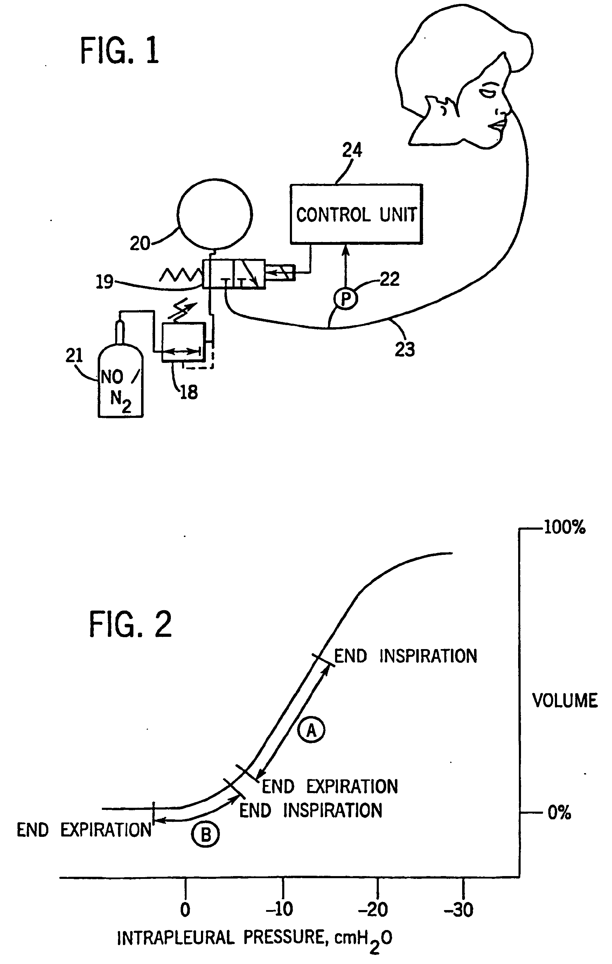

A device for pulsed NO administration is shown in Fig. 1. The NO from the supply 21 is conducted through a pressure regulator 18, regulating the supply pressure to an appropriate level, and through a delivery control valve 19 to a dosing chamber 20. The dosing chamber has a fixed known volume. When loading into fixed known volume at a pressure regulated by the pressure regulator 18, the amount of gas in the dosing chamber is known. For delivery, the control unit 24 activates the control valve 19 connecting the dosing chamber to the delivery line 23 and closing the connection between the NO supply and the dosing chamber. The gas from the dosing chamber will be exhausted until the dosing chamber pressure equilibrates with the environmental pressure. The gas delivered is thus the difference in the dosing chamber gas amount between the beginning and end of the delivery pulse. The dosing chamber volume depends on the volume to be delivered. As an example, the 50 ml pulse volume used in clinical trials with horses, could be generated with 1 bar regulator overpressure and 50 ml chamber volume, or alternatively 2 bar overpressure and 25 ml chamber volume. With fixed chamber volume, adjusting the regulated supply pressure can regulate the dose volume. Alternatively, the valve opening can be of fixed duration. For a subject breathing in the normal manner, the NO pulse may be delivered to the subject by a nasal cannula, or other suitable means.

The dose is administered through delivery line 23 to the nasal cannula. For synchronization of the pulse administration, the NO delivery device is equipped with a pressure sensor. Inspiration by the subject causes an under pressure in the spontaneous breathing. This pressure signal is transported through the delivery line to a pressure sensor 22. When the sensor detects triggering pressure conditions, the control unit 24 activates the valve 19 for dose delivery. The same pressure sensor can monitor the delivery since the flow resistance of the delivery line causes a pressure increment at the measurement point. After the pulse, the valve 19 is deactivated reestablishing the connection between the NO supply and the dosing chamber for dosing chamber reload.

As noted above, the present invention is directed to the provision of NO to subjects having essentially healthy lungs but for various reasons are suffering from a lack of adequate oxygenation of the blood. Inadequate oxygenation is evidenced by a large alveolar-arterial oxygen partial pressure difference or by a low arterial oxygen partial pressure level. With respect to the alveolar-arterial oxygen partial pressure difference, a difference larger than 20 kPa is evidence of inadequate oxygenation. In normal human subjects, the difference is essentially zero. With respect to arterial oxygen partial pressures, a partial pressure of less than 10 kPa is definite evidence of inadequate oxygenation in humans, the normal level being 13 kPa when breathing air. The administration of NO can be based on a determination that one or both of these conditions exist in the subject.

Fig. 2 presents the volume change in different lung sections at various intrapleural pressures encountered in the course of spontaneous breathing. The designation A shows alveoli remaining open throughout the expiration and the designation B shows alveoli collapsing during expiration and opening during inspiration. The arrows in the designations indicate the range of volumetric movement of the respective alveoli in the volume-intrapleural pressure curve. The figure shows the temporary opening of the alveoli identified as B. The NO delivery into these alveoli should be limited below the amount needed for vasodilatation of the capillaries proximate to these alveoli, since during expiration there is no alveolar volume remaining for gas exchange, and the increased blood flow increases the shunt. The alveoli identified as A remain open throughout the breath cycle and should be provided NO sufficient for inducing the vasodilatation.

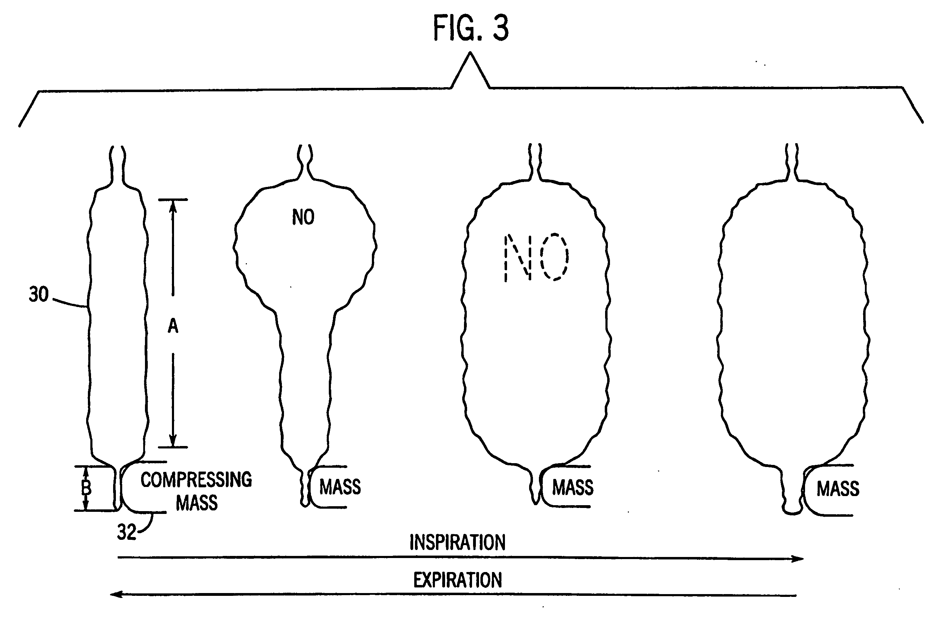

Fig. 3 shows the same information as Fig. 2 in simplified form in which the portion of a healthy lung 30 containing alveoli B is shown compressed by tissue mass 32. As inspiration proceeds from left to right in Fig. 3, breathing gases containing NO, for example as a pulsatile dose, inflate lung 30. The inflation commences with the upper portion of the lung, i.e. the portion containing alveoli A shown in Fig. 2. By the time the end of inspiration approaches, as in the right hand side of Fig. 3, the concentration of NO has been diluted by the inhaled breathing gases and/or removed by gas transfer in alveoli A, so that little or no NO remains as the compressed portion of the lung finally inflates. NO delivery to alveoli B is thus limited. Fig. 3 also shows the action of the lung upon expiration.

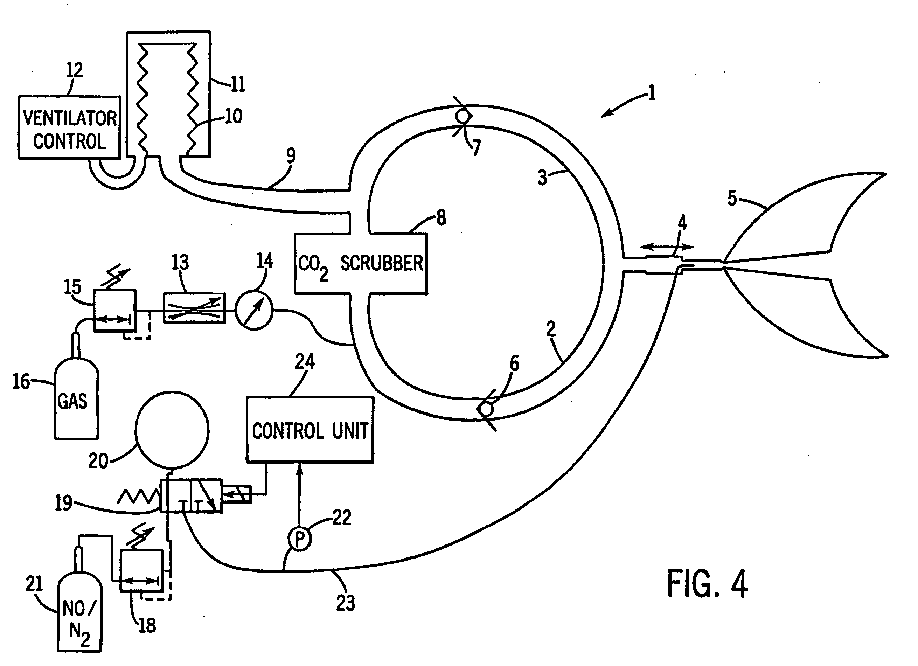

A device for pulsed NO administration is also disclosed in earlier patent of a co-inventor named in this application, U.S. Patent 5,918,596. An alternate delivery apparatus specifically designed for NO administration in pulses synchronously with the inspiration is presented in Fig. 4 in the setting of an anesthesia system.

A particular advantage of pulsed NO delivery is obtained when a rebreathing circuit of this type shown in Fig. 4 is used. NO reacts with oxygen forming nitrogen dioxide (NO₂). NO₂ is highly poisonous gas and the concentration should be kept below 2 ppm (OHSA limit for working environment). NO₂ formation rate depends on the reaction time available, the concentration of oxygen present and the square of NO concentration. Delivering NO as a short pulse synchronously with inspiration directs the NO into perfused alveoli. Due to the high diffusion constant of NO into blood, the alveolar NO is rapidly taken up, and only a minor fraction will be exhaled to the breathing circuit keeping the circuit concentration low, thus reducing the formation of NO₂. With constant inspired concentration delivery, NO is administered into anatomic dead spaces as well as the lungs. When used with a breathing circuit, all the dead space gas will be exhausted, increasing the breathing circuit NO concentration and thereby the NO₂ formation.

In the NO therapy system presented in Fig. 4, the patient breathing circuit 1 is comprised of inspiratory limb 2, expiratory limb 3, subject limb 4, one-way valves 6 and 7, CO₂ scrubber 8, and ventilator lime 9. The breathing circuit is of standard construction and connects the subject's lungs 5 with the ventilator connecting tube 9. The ventilator connecting tube 9 connects the breathing circuit to a bellows 10 into which the subject expires during expiration. The bellows may be located within a container 11 connected to ventilator control 12 for artificial ventilation of the subject. One way valves 6 and 7 direct the inspiration and expiration flows to the respective flow paths. The scrubber 8 removes the CO₂ from the expired gas during rebreathing. The CO₂ cleaned gas is supplied with fresh gas from a gas mixer comprising a fresh gas flow control 13 and metering 14 for controlling the gas flow from supply 16. The pressure regulator 15 reduces the supply pressure to an appropriate level for the flow control. The fresh gas supply may comprise multiple gas sources and may include an anaesthetic vaporizer. The dose is administered through delivery line 23 to the subject limb 4 of the breathing circuit. In the embodiment shown in Fig. 4, pressure sensor 22 senses the over pressure produced by bellows 10.

In a clinical trial of 6 horses anaesthetized with isoflurane in oxygen in supine position, NO pulse volumes of 3.6 µmol and 4.9 µmol were delivered for each inspiration both in spontaneous breathing and in artificial ventilation. The NO delivery was carried out in the manner described above in which the delivery of NO was limited in the alveoli collapsing during expiration and opening during inspiration due to the supine position of the horses. Following the delivery, the arterial oxygen partial pressure increased from mean 14.5 kPa (5.1 kPa standard deviation) to 28.1 kPa (11). The change in pulmonary shunt calculated from the arterial and venous oxygen contents with the shunt equation was from 32.2% (5.9) to 22.4 (6.0). The changes were statistically significant (p<0.01). In contrast to this, 10 ppm constant inspired concentration did not provide any improvement in another study (Young et al.: "*Effects of inhaled nitric oxide 10 ppm in spontaneously breathing horses anaesthetized with halothane*", British Journal of Anaesthesia, August 1999).

As noted above, Figs. 1 and 4 show the pulsed administration of NO. The invention may be practiced using a constant NO concentration during inspiration. In such a case a gas flow control, similar to that shown as 13 in Fig. 4 may be provided between the pressure regulator for the NO supply and a delivery line to control the amount of NO supplied to the subject during inspiration.

It is recognized that other equivalents, alternatives, and modifications aside from those expressly stated, are possible and within the scope of the appended claims.

## Claims

1. Use of NO gas for the preparation of a breathable gas for improving oxygenation and reducing shunt perfusion by providing NO gas into ventilated alveoli of the lungs in a subject having no diagnosis of lung injury but suffering impaired oxygenation, the hmgs of the subject having a ventilated portion of alveoli that remain open during inspiration and expiration and a further alveolar portion comprising one in which the alveoli collapse during expiration as a result of compression by tissue mass, wherein the step of providing NO gas to the lungs of the subject is carried out in such a manner that a sufficient amount of NO is delivered to the ventilated alveolar portion to cause capillary vasodilatation while providing an amount of NO to the further alveolar portion which is small compared to that delivered to said ventilated alveolar portion.

2. Use according to claim 1, wherein an amount of NO is delivered to the further alveolar portion which is less than that which induces capillary vasodilatation in the alveoli of the further portion.

3. Use according to claim 1 or 2 wherein the concentration of the NO in the breathing gases of the subject is controlled to a level, which when inhaled, will achieve the desired provision of NO to the ventilated and further alveolar portions of the lung.

4. Use according to any of claims 1 to 3, wherein the NO gas is administered in a pulse dose in the inspired breathing gases of the subject and wherein at least one of the timing and duration of the pulse dose is controlled to achieve the desired delivery of the NO.

5. Use according to claim 4, wherein the pulse dose is provided during the first 70% of the time during which the subject is inspiring.

6. Use according to claim 5, wherein the pulse dose is provided during the first half of the time the subject is inspiring.

7. Use according to any of claims 1 to 3, wherein the NO gas is continuously provided into the breathing gases of the subject.

8. Use according to claim 7, wherein the delivery rate of NO into the breathing gases of the subject is controlled.

9. Use according to any of claims 1 to 8, wherein the subject's alveolar-arterial oxygen partial pressure difference is measured and NO gas is provided when the pressure difference is above a predetermined threshold.

10. Use according to claim 9, wherein NO gas is provided when the partial pressure difference is greater than 20 kPa.

11. Use according to any of claims 1 to 8, wherein the subject's arterial oxygen partial pressure is measured and NO gas is provided when the partial pressure is less than a predetermined threshold.

12. Use according to claim 11, wherein NO gas is provided when the arterial oxygen partial pressure is less than 10 kPa.

13. Use according to any preceding claim, wherein the subject is an equine subject in a supine position.

14. Use according to any of claims 1 to 12, wherein the subject is an obese human subject whose obesity reduces the functional residual capacity of the lungs of the subject.

## Patentansprüche

1. Verwendung von NO-Gas zur Herstellung eines atembaren Gases zur Verbesserung der Oxygenierung und Reduktion der Shuntperfusion durch Bereitstellung von NO-Gas in ventilierte Alveolen der Lungen in einem Patienten mit keiner Diagnose einer Lungenverletzung, der aber an einer beeinträchtigten Oxygenierung leidet, wobei die Lungen des Patienten einen ventilierten Anteil der Alveolen aufweisen, die während der Ein- und Ausatmung offen bleiben, und einen weiteren alveolären Anteil, umfassend einen, worin die Alveolen während der Ausatmung aufgrund der Kompression durch Gewebsmasse kollabieren, worin der Schritt des Bereitstellens von NO-Gas an die Lungen des Patienten auf eine derartige Weise durchgeführt wird, dass eine ausreichende NO-Menge an den ventilierten alveolären Anteil abgegeben wird, um die Vasodilatation der Kapillaren herbeizuführen, während eine NO-Menge an den weiteren alveolären Anteil bereitgestellt wird, die im Vergleich zu der, die an den genannten ventilierten alveolären Anteil abgegeben wird, klein ist.

2. Verwendung nach Anspruch 1, worin eine NO-Menge an den weiteren alveolären Anteil abgegeben wird, die geringer ist als die, welche die Vasodilatation der Kapillaren in den Alveolen des weiteren Anteils induziert.

3. Verwendung nach Anspruch 1 oder 2, worin die Konzentration des NO in den Atemgasen des Patienten auf eine Höhe kontrolliert wird, die - wenn inhaliert - die gewünschte Versorgung mit NO an die ventilierten und weiteren alveolären Anteile der Lunge erreicht.

4. Verwendung nach einem der Ansprüche 1 bis 3, worin das NO-Gas in einer Pulsdosis in den eingeatmeten Atemgasen des Patienten verabreicht wird und worin mindestens eine von der zeitlichen Festlegung und der Dauer der Pulsdosis zum Erreichen der gewünschten Abgabe des NO kontrolliert wird.

5. Verwendung nach Anspruch 4, worin die Pulsdosis während der ersten 70 % der Zeit, während welcher der Patient einatmet, bereitgestellt wird.

6. Verwendung nach Anspruch 5, worin die Pulsdosis während der ersten Hälfte der Zeit, während welcher der Patient einatmet, bereitgestellt wird.

7. Verwendung nach einem der Ansprüche 1 bis 3, worin das NO-Gas kontinuierlich in die Atemgase des Patienten bereitgestellt wird.

8. Verwendung nach Anspruch 7, worin die Abgaberate von NO in die Atemgase des Patienten kontrolliert wird.

9. Verwendung nach einem der Ansprüche 1 bis 8, worin die Differenz des alveolär-arteriellen Sauerstoffpartialdrucks gemessen wird und NO-Gas bereitgestellt wird, wenn die Druckdifferenz über einer prädeterminierten Schwelle liegt.

10. Verwendung nach Anspruch 9, worin NO-Gas bereitgestellt wird, wenn die Partialdruckdifferenz mehr als 20 kPa beträgt.

11. Verwendung nach einem der Ansprüche 1 bis 8, worin der arterielle Sauerstoffpartialdruck des Patienten gemessen wird und NO-Gas bereitgestellt wird, wenn der Partialdruck unter einer prädeterminierten Schwelle liegt.

12. Verwendung nach Anspruch 11, worin NO-Gas bereitgestellt wird, wenn der arterielle Sauerstoffpartialdruck weniger als 10 kPa beträgt.

13. Verwendung nach einem der vorangehenden Ansprüche, worin der Patient einen equinen Patienten in Rückenlage darstellt.

14. Verwendung nach einem der Ansprüche 1 bis 12, worin der Patient einen adipösen humanen Patienten darstellt, dessen Adipositas die funktionelle Residualkapazität der Lungen des Patienten reduziert.

## Revendications

1. Utilisation de gaz de monoxyde d'azote pour la préparation d'un gaz respirable destiné à améliorer l'oxygénation et réduire le shunt de perfusion en fournissant du gaz de monoxyde d'azote aux alvéoles pulmonaires ventilées d'un sujet qui n'a pas de diagnostic de blessure pulmonaire mais qui souffre d'une oxygénation défectueuse, les poumons du sujet ayant une portion ventilée d'alvéoles qui restent ouvertes durant l'inspiration et l'expiration et une autre portion alvéolaire qui comprend une portion dans laquelle les alvéoles s'affaissent durant l'expiration à cause de la compression d'une masse tissulaire, selon laquelle l'étape consistant à fournir du gaz de monoxyde d'azote aux poumons du sujet est effectuée de manière à délivrer une quantité suffisante de monoxyde d'azote à la portion alvéolaire ventilée pour provoquer une vasodilatation capillaire tout en fournissant une quantité de monoxyde d'azote à l'autre portion alvéolaire qui est plus petite comparée à ce qui est délivré à ladite portion alvéolaire ventilée.

2. Utilisation selon la revendication 1, selon laquelle une quantité de monoxyde d'azote est délivrée à l'autre portion alvéolaire qui est inférieure à celle qui déclenche une vasodilatation capillaire dans les alvéoles de l'autre portion.

3. Utilisation selon la revendication 1 ou 2 selon laquelle la concentration du monoxyde d'azote dans les gaz de respiration du sujet est régulée jusqu'à un niveau qui, lorsqu'inhalé, apportera la quantité souhaitée de monoxyde d'azote aux portions ventilées et aux autres portions alvéolaires du poumon.

4. Utilisation selon l'une quelconque des revendications 1 à 3, selon laquelle le gaz de monoxyde d'azote est administré en une dose pulsée dans les gaz de respiration inspirés par le sujet et selon laquelle au moins soit le moment soit la durée d'administration de la dose pulsée est régulée pour obtenir la quantité délivrée souhaitée de monoxyde d'azote.

5. Utilisation selon la revendication 4, selon laquelle la dose pulsée est fournie dans les premiers 70 % du temps durant lequel le sujet inspire.

6. Utilisation selon la revendication 5, selon laquelle la dose pulsée est fournie dans la première moitié du temps durant lequel le sujet inspire.

7. Utilisation selon l'une quelconque des revendications 1 à 3, selon laquelle le gaz de monoxyde d'azote est continuellement fourni dans les gaz de respiration du sujet.

8. Utilisation selon la revendication 7, selon laquelle le débit du gaz de monoxyde d'azote délivré dans les gaz de respiration du sujet est régulé.

9. Utilisation selon l'une quelconque des revendications 1 à 8, selon laquelle la différence de pression partielle en oxygène alvéolaire-artériel du sujet est mesurée et du gaz de monoxyde d'azote est fourni quand la différence de pression est au-dessus d'un seuil prédéterminé.

10. Utilisation selon la revendication 9, selon laquelle du gaz de monoxyde d'azote est fourni quand la différence de pression partielle est supérieure à 20 kPa.

11. Utilisation selon l'une quelconque des revendications 1 à 8, selon laquelle la pression partielle en oxygène artériel du sujet est mesurée et du gaz de monoxyde d'azote est fourni quand la pression partielle est inférieure à un seuil prédéterminé.

12. Utilisation selon la revendication 11, selon laquelle du gaz de monoxyde d'azote est fourni quand la pression partielle en oxygène artériel est inférieure à 10 kPa.

13. Utilisation selon l'une quelconque des revendications précédentes, selon laquelle le patient est un sujet chevalin en position couchée.

14. Utilisation selon l'une quelconque des revendications 1 à 12, selon laquelle le patient est un sujet humain dont l'obésité réduit la capacité résiduelle fonctionnelle des poumons du sujet.
